(19) Europäisches Patentamt
European Patent Office
Office européen des brevets

(11) Publication number: **0 063 946**
**A1**

(12) # EUROPEAN PATENT APPLICATION

(21) Application number: 82302132.4

(22) Date of filing: 26.04.82

(51) Int. Cl.³: **A 61 K 49/02**, A 61 K 43/00, C 07 C 149/20, C 07 C 163/00

(30) Priority: 27.04.81 GB 8112866

(43) Date of publication of application: 03.11.82 Bulletin 82/44

(84) Designated Contracting States: DE FR GB

(71) Applicant: AMERSHAM INTERNATIONAL plc, White Lion Road, Amersham Buckinghamshire, HP7 9LL (GB)

(72) Inventor: Nowotnik, David, AMERSHAM INTERNATIONAL plc White Lion Road, Amersham Buckinghamshire, HP7 9LL (GB)

(74) Representative: Pennant, Pyers et al, Stevens, Hewlett & Perkins 5 Quality Court Chancery Lane, London, WC2A 1HZ (GB)

(54) **Diagnosis of kidney function.**

(57) A complex of Technetium-99m with an acid having the formula $HOOC-CH_2-X-CH_2-COOH$, where X is sulphur or selenium has glomerular filtration and tubular secretion properties which make it useful in the diagnostic technique of renography to give a dynamic representation of the functioning of the kidneys. The complex may be prepared a) by reducing $TcO_4^-$ with a $Sn^{++}$ reducing agent in the presence of $Cu^+$ and of the acid, or b) by reducing $TcO_4^-$ with a tin metal reducing agent in the presence of a cupric salt and of the acid.

EP 0 063 946 A1

ACTORUM AG

# DIAGNOSIS OF KIDNEY FUNCTION

The well established nuclear medicine diagnostic technique of renography gives a dynamic representation of the removal of substances from the blood by the kidneys, and their excretion into the urine. A radioactive material which is rapidly excreted from the kidneys is injected into the patient, and its passage through the kidneys is observed with detectors placed over the kidneys (probe renography). The current material of choice for this purpose is sodium iodohippurate ("Hippuran") I-131.

Instead of using a single detector over each kidney, it is possible to use a gamma camera. Not only is this much more convenient, especially with children, since accurate positioning is no longer essential, but the results can be stored on tape and the tape replayed in such a way as to accept only the signals from a selected area of the kidney. For this and other reasons, the technique of gamma camera renography is potentially a very powerful one.

Development of the technique has however been held up by the difficulty of finding a suitable radioactive

BAD ORIGINAL

material for injection into the patient. A gamma camera renogram requires much larger quantities of activity than does a simple probe renogram, and, with iodine-131, the radiation dose to the patient (especially one with impaired renal function) would be quite unacceptable. For many years, therefore, workers in the field have attempted to find a suitable Tc-99m complex which could be used in place of Hippuran I-131. This invention results from the discovery of such a substance.

The kidney contains about 1 million nephrons, each comprising a glomerulus and a tubule. The glomerulus acts as a molecular filter, by allowing water from the blood to pass through (this is the source of the urine) together with all small molecules; it will not allow the passage of large protein molecules such as albumin. The tubules re-absorb most of the water from the filtrate back into the blood, and re-absorb essential small molecules such as glucose; they also allow other small molecules to pass from the blood to the urine (tubular secretion).

Of the blood flowing through the kidney, about 20% of the water present passes through the glomerular filter. Thus, if a substance is removed from the blood only by glomerular filtration, only 20% of it will be removed from the blood in a single pass through the kidney. However, if it is removed effectively also by tubular secretion, up to 100% of it may be removed from the blood in a single pass.

To obtain a renogram with good diagnostic properties, as much as possible of the radioactive substance should be removed in a single pass. About 80% of Hippuran is removed in a single pass. Other substances are known

which are removed as efficiently as Hippuran, but like Hippuran they cannot be labelled with technetium - 99m.

An example of substances which are removed from the blood by glomerular filtration only are metal complexes of diethylene triamine pentacetic acid (DTPA). DTPA is easily labelled with technetium -99m, and the labelled product has been used for gamma camera renograms. But DTPA -Tc-99m shows a clearance rate only about 20% of that of Hippuran -I-131, and as a result, renograms obtained with DTPA -Tc-99m are flat and of limited diagnostic value. A large number of substances are known which are removed from the blood by glomerular filtration only, and many of these could be labelled with technetium -99m, but renograms obtained using these substances would suffer from the same disadvantages as those obtained from DTPA.

Three necessary features for a gamma camera renography agent are:-

a) it must be capable of being removed from the blood by tubular secretion as well as by glomerular filtration in the kidneys;

b) it must not accumulate significantly in the kidney or any other organ; and

c) it must be capable of being labelled with Technetium -99m.

The present invention results from our discovery that substances having the aforesaid characteristics are acids having the formula

$$HOOC - CH_2 - X - CH_2 - COOH$$

where X is sulphur or selenium and non-toxic salts thereof. These substances are denoted as thiodiglycollic acid (TDG) and its selenium analogue. Experiments in pigs have shown

TDG - Tc-99m to have a clearance rate 59% of that of Hippuran - I-131, thus demonstrating that TDG is indeed removed by tubular secretion.

TDG -Tc-99m is a known compound, for it has been described by Inoue O, Ikeda I and Kurata K in Radioisotopes, 1976, 25, 24 - 30. The authors were looking for agents for renal scanning, that is to say, for static agents which would give a high kidney to background emission ratio. For this purpose, they required agents which would become localised in the kidneys. Although there is some confusion between TDG and DTG (dithiodiglycollic acid) in the article, their conclusions were that TDG -Tc-99m was excreted rapidly outside the kidneys; and appeared to behave similarly to DTPA -Tc-99m; and was therefore unsuitable for their purpose.

Inoue et al were not concerned with glomerular filtration and tubular excretion. Their article does not suggest that TDG- Tc-99m might be excreted by tubular excretion, and accordingly does not suggest that this agent might be suitable for gamma camera renography.

In one aspect, the present invention is for the use of TDG -Tc-99m (or its selenium analogue) in renography.

In another aspect, this invention provides, in a method of performing renography by administering to a patient a radioactive substance which accumulates transiently in the kidneys, but is rapidly excreted from them into the urine without significant accumulation elsewhere in the body, mounting a gamma camera or other device for recording gamma-radiation adjacent each kidney, recording the variations in radioactive emissions from each kidney with time and comparing the records so as to detect malfunctioning of either kidney, the improvement which consists in that the radioactive substance used is TDG-Tc-99m or its selenium analogue.

In another aspect, this invention relates to the preparation of TDG-Tc-99m and its selenium analogue. Technetium -99m is obtained in the eluate from a technetium generator as the pertechnetate ion $TcO_4^-$, in which form it does not readily form complexes. The technetium is therefore reduced from the 7+ valency, probably to the 3+. 4+ or 5+ valency and often in the presence of the reagent with which it is desired to form a complex. Various methods involving tin as the reducing agent are known for this purpose, but not all are equally effective to form a complex having the desired biological properties. This invention envisages two alternative methods:-

i) The conventional method for the production of Tc-99m complexes, namely addition of the generator eluate to a large excess of $Sn Cl_2$ or other water-soluble stannous salt in admixture with TDG gives rise to a product having relatively poor biological properties. Mixtures of $Sn Cl_2$ or other stannous salt together with a cuprous or cupric salt were found to give rapid formation of a product having good biological properties.

ii) When tin metal, rather than a stannous salt, is used as the reducing agent, the reduction proceeds rather slowly and incompletely and an insoluble residue is produced. However, when a water-soluble salt of a more noble metal than tin, for example cupric chloride $CuCl_2$, is present, the reduction goes rapidly with little formation of solid residue to give a product having satisfactory biological properties.

The following Examples show preparation of the labelled complexes.

EXAMPLE 1

Cuprous chloride (50mg) was dissolved in concentrated hydrochloric acid (5ml), and this solution added to a stirred solution of TDG (1.0g) and $SnF_2$ (2mg) in nitrogen-purged water (95ml). The pH of the resultant solution was adjusted to 4.0 with a concentrated solution of sodium hydroxide and the volume increased to 100ml with water. (TDG is about half neutralised at pH4). 1ml aliquots were dispensed into 10ml glass vials. Formation of the Tc-99m complex was achieved either by direct treatment of the TDG solution with a saline solution of $TcO_4^-$, or by treatment of the solid resulting from freeze drying the TDG solution with a saline solution of $TcO_4^-$.

Each vial contained ingredients derived from:

| | |
|---|---|
| TDG | 10mg |
| $SnF_2$ | 20µg |
| CuCl | 500µg |

Formation of the desired complex was rapid and the biological results were satisfactory.

However, with the freeze dried ampoules, some variability of results indicated that the preparation was insufficiently robust to freeze drying.

Similar results were obtained when cupric chloride was used in place of cuprous chloride.

EXAMPLE 2

A TDG solution was prepared and dispensed as described in Example 1, except that 1.5ml of the cuprous chloride in HCl solution was added to a solution of TDG (1.0g), $SnF_2$ (50mg), and NaF (100mg) in water 95ml). Formation of the Tc-99m complex was also as described in 1.

Each vial contained ingredients derived from:

| | |
|---|---|
| TDG | 10mg |
| $SnF_2$ | 500µg |
| NaF | 1mg |
| CuCl | 150µg |

Formation of the desired complex was rapid and the biological results were satisfactory.

The preparation proved robust to freeze drying.

EXAMPLE 3

640 mg of recrystallised TDG was dissolved in 28 ml deionised water, and 4 ml of a 4 mg/ml solution of $CuCl_2.2H_2O$ was added. The pH of the solution was adjusted to 4.0 with solid $NaHCO_3$, or with dilute NaOH solution. The solution was filtered and 1 ml aliquots dispensed in vials and freeze-dried. $1cm^2$ pieces of tin foil were degreased by washing with methyl ethyl ketone. One piece of foil was added to each vial, and the vial purged with nitrogen and sealed. Each vial contained

| | |
|---|---|
| TDG | 20mg |
| $CuCl_2.2H_2O$ | 0.5mg |
| Tin foil | $1.cm^2$ |
| | pH 4.0 |

Each vial was reconstituted by the addition of 15ml of a saline solution of $TcO_4^-$ and the desired complex was rapidly formed.

The selenium analogue of TDG - Tc-99m may be made analogously by using selena diglycollic acid in place of TDG

EXAMPLE 4

Selenadiglycollic acid (SeDG) was prepared as described in the literature (J.C.S. Dalton Trans., 1975,

2297). Preparation of the technetium complex was as follows:-

A $1cm^2$ piece of tin foil was "Activated" by treatment with 10ml of concentrated hydrochloric acid for 1 minute. The tin foil was thoroughly washed with ethanol, and placed in a 10ml glass vial.

Added to the vial were 0.5ml of freshly prepared solution of SeDG (prepared at a concentration of 4mg/ml, with the pH adjusted to 4.0 using solid $NaHCO_3$), and 0.5ml of a saline solution of $TcO_4^-$. Formation of the Tc complex was slow, but yields in excess of 92% were attained 1 hour postpreparation.

Samples for animal biodistribution studies were administered 1-2 hours post preparation. In rats, Tc-99m SeDG demonstrated a mean clearance half time of 14.6 minutes and >90% of injected activity in the bladder on dissection, 2 hours post administration,

EXAMPLE 5

ANIMAL TESTS IN PIGS.

Female pigs were anaesthetized and positioned in front of two radiation detection probes, one placed over the right kidney and the other over the heart. Into an ear vein was injected one of the two following solutions:-

A TDG - Tc-99m and
Hippuran - I-131

B DTPA - Tc-99m and
Hippuran - I-131

The probes recorded radioactive emissions from both Tc-99m and separately I-131 over a period of 80 minutes after injection, and typical results (which are means of readings from several animals) are set out in Table I.

TABLE I

| Test | Solution | |
|---|---|---|
| | A | B |
| Relative kidney uptake index | | |
| 5 minutes | 53.3 | 32.1 |
| 10 minutes | 51.7 | 26.8 |
| 30 minutes | 59.5 | 38.2 |
| Kidney counts | | |
| Time to max (min.) | 1.13 | 1.15 |
| Time to ½ max (min.) | 1.32 | 2.17 |
| 80 min as % of max | 1.91 | 3.93 |
| Heart clearance ratio (Tc/I) | 1.75 | 3.46 |

The relative kidney uptake index is a measure of the kidney to background ratio of the Tc agent compared with the corresponding ratio of Hippuran at different times after injection

$$\frac{\text{Tc kidney} \div \text{Tc heart}}{\text{Hippuran kidney} \div \text{Hippuran heart}} \times 100\%$$

The ratio for TDG at maximum is 1.6 to 2.0 times greater than for DTPA.

The kidney counts are for Tc-99m only and show the time taken after injection to reach maximum count rate; the time taken after injection for the count rate to fall to half the maximum; and the count rate at 80 minutes as a percentage of the maximum. Significantly more of the DTPA complex remains in the kidney 80 minutes

after injection than of the TDG complex.

The heart clearance ratio is the time taken for half the Tc-99m radioactivity to be cleared from the heart expressed as a fraction of the time taken for half the I -131 radioactivity to be cleared from the heart. TDG was cleared significantly more quickly than was DTPA. In fact, the clearance rate of TDG was 59% of Hippuran, and that of DTPA was 28% of Hippuran.

DTPA is a representative substance which is cleared from the blood by glomerular filtration but not by tubular secretion. The above results demonstrate that TDG is cleared from the blood by both glomerular filtration and tubular secretion and does not accumulate significantly in the kidney or in any other organ.

## EXAMPLE 6

## ANIMAL TESTS IN RATS.

Comparative data for Hippuran, Tc-99m TDG and Tc-99m DTPA in rats is set out in Table II.

TABLE II

| Agent | 2 hour dissection (% injected dose) | | | | % in urine at 30 min p.i. | Clearance half time (min) |
|---|---|---|---|---|---|---|
| | Kidney | Liver | Intestine | Bladder | | |
| Hippuran | 0.2 | 0.2 | 2.5 | 93.0 | 82.6 | 10.4 |
| Tc-DTPA | 1.0 | 0.4 | 0.9 | 96.0 | 66.5 | 17.1 |
| Tc-DTG | 0.5 | 1.2 | 1.0 | 94.5 | 73.7 | 13.5 |

## CLAIMS

1. A complex of an acid having the formula

$$HOOC - CH_2 - X - CH_2 - COOH,$$

where X is sulphur or selenium, or a non-toxic salt thereof, with technetium-99m, for usein renography.

2. A method of making the complex defined in claim 1, which method comprises reducing $TcO_4^-$ with a $Sn^{++}$ reducing agent in the presence of $Cu^+$ or $Cu^{2+}$ and of an acid having the formula $HOOC - CH_2 - X - CH_2 - COOH$, where X is sulphur or selenium or a non-toxic salt thereof, and recovering the formed complex.

3. A method of making the complex defined in claim 1, which method comprises reducing $TcO_4^-$ with a tin metal reducing agent, in the presence of a water-soluble salt of a more noble metal than tin and of an acid having the formula $HOOC - CH_2 - X - CH_2 - COOH$, where X is sulphur or selenium, or a non-toxic salt thereof, and recovering the formed complex.

4. A method as claimed in claim 3, wherein the water-soluble salt of a more noble metal than tin is a cupric salt.

5. A complex of an acid having the formula

$$HOOC - CH_2 - Se - CH_2 - COOH$$

or a non-toxic salt thereof, with technetium-99m.

European Patent Office

# EUROPEAN SEARCH REPORT

0063946
Application number

EP 82 30 2132

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | CHEMICAL ABSTRACTS, vol. 85, no. 1, 5th July 1976<br>abstract no. 1835t, page 160<br>COLUMBUS, OHIO (US)<br>& RADIOISOTOPES 1976, 25(1), 24-30<br>I. INOUE et al.: "Technetium-99m-labeled mercapto compounds for renal scanning"<br>* abstract * | 1-5 | A 61 K 49/02<br>43/00<br>C 07 C 149/20<br>163/00 |
| | --- | | |
| A | FR - A - 2 048 610 (SCHERING)<br>* page 8, line 5 * | 5 | |
| | --- | | |
| A | DE - A - 2 419 310 (MINNESOTA MINING)<br>* page 10, lines 5-9 * | 1-5 | TECHNICAL FIELDS SEARCHED (Int. Cl. 3) |
| | --- | | A 61 K 49/00<br>43/00<br>C 07 C 149/00<br>163/00 |
| A | FR - A - 2 269 354 (SOLCO BASEL)<br>* page 17, claim 1 * | 1-5 | |
| | --------- | | |

The present search report has been drawn up for all claims

| Place of search | Date of completion of the search | Examiner |
|---|---|---|
| The Hague | 16-06-1982 | GRAMAGLIA |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document